# EUROPEAN PATENT SPECIFICATION

(11) **EP 2 886 119 B1**
(45) Date of publication and mention of the grant of the patent: **02.11.2016**
(21) Application number: 13830269.0
(22) Date of filing: 15.08.2013
(51) Int. Cl.: A61K 31/473, A61K 9/22, A61K 9/48, A61P 15/10, A61K 47/38, A61K 47/02, A61K 47/12, A61K 9/20

(54) **PHARMACEUTICAL COMPOSITIONS CONTAINING IPIDACRINE AND USE THEREOF TO TREAT POTENCY DISORDERS AND OTHER SEXUAL ACTIVITY DISORDERS**
PHARMAZEUTISCHE ZUSAMMENSETZUNGEN MIT IPIDACRIN UND VERWENDUNG DAVON ZUR BEHANDLUNG VON POTENZSTÖRUNGEN UND ANDEREN STÖRUNGEN DER SEXUELLEN AKTIVITÄT
COMPOSITIONS PHARMACEUTIQUES CONTENANT DE L'IPIDACRINE ET LEUR UTILISATION POUR TRAITER DES TROUBLES DE PUISSANCE SEXUELLE OU D'AUTRES FORMES DE L'ACTIVITÉ SEXUELLE

(30) Priority: 20.08.2012 RU 2012135579
(43) Date of publication of application: 24.06.2015
(73) Proprietor: Limited Liability Company "konsortsium-pik", Moscow 125047 (RU)
(72) Inventor: BYKOV, Vladimir Nikolaevich, St.Petersburg 194017 (RU); NIKIFOROV, Aleksandr Sergeevich, St.Petersburg 197371 (RU); KIM, Galina Aleksandrovna, Moscow 117570 (RU)
(74) Representative: Carvajal y Urquijo, Isabel
(86) International application number: PCT/RU2013/000707
(87) International publication number: WO 2014/031034

(56) References cited:
- WO-A1-02/051416
- JP-A- 2002 255 820
- US-B1- 6 433 173
- 'Amiridin 20 mg. Reg. N. P N011762/01-2000' OLAINFARM 19 December 2013, LATVIYA, XP055178333 Retrieved from the Internet: <URL:http://www.rlsnet.ru/prep_index_id_418 47.htm> [retrieved on 2013-12-19]
- 'Promyshlennaya tekhnologiya lekarstv: uchebnik, v 2-kh t. Tom 2.' POD RED. CHUESHOVA. -X.: MTK-KNIGA. IZDATELSTVO NFAU vol. 716, 2002, pages 331 - 332, XP008175633

## Description

### Field of the invention

The invention relates to medicine, namely to endocrinology, andrology, in particular, the invention refers to the use of ipidacrine for the treatment of disorders of potency and other forms of sexual activity.

### Background of the invention

Some pharmaceutical formulations containing ipidacrine (Axamon, Amiridine, Neyromidin) are well known, but these formulations are intended for the use as drug products for the treatment of other diseases, namely diseases of the peripheral nervous system (PNS) including (mono-and polyneuropathy) polyradiculopaty, myasthenia and Eaton-Lambert syndrome of various etiologies); diseases of the central nervous system (CNS), including bulbar paralysis and paresis; for the use in the recovery period in case of organic lesions of the central nervous system accompanied by motor and/or cognitive impairment); intestinal atony (treatment and prevention).

Therefore, the use of pharmaceutical formulations containing ipidacrine for the treatment of disorders of potency and other forms of sexual activity is proposed by the authors for the first time.

### Objects of the invention

The invention can be applied in the clinic for the treatment of potency disorders associated with the decreased production of hormones by the sex glands; disorders caused by chronic (including physical) stress as well as against the background of spontaneously reduced sexual function including manifested anorgasmia or delayed ejaculation, other disorders of sexual activity which do not limit the scope of the invention.

The object of the present invention is to extend the use of ipidacrine for the treatment of sexual dysfunction associated with the decreased production of hormones by the sex glands, chronic stress as well as manifested anorgasmia or delayed ejaculation as well as the creation of a new pharmaceutical formulation containing ipidacrine which would expand the use of ipidacrine in pharmaceutical formulations and provide the use of ipidacrine in pharmaceutical formulations for the treatment of sexual activity disorders associated with reduced production of hormones by the sex glands; disorders caused by chronic (including physical) stress as well as against the background of the spontaneously reduced sexual function including manifested anorgasmia or delayed ejaculation.

### Summary of the invention

Ipidacrine is a reversible cholinesterase inhibitor. Four classes of cholinesterase inhibitors are known (aminopyridines, organophosphates, carbamates and all the rest). Ipidacrine is an aminopyridine that possesses the structural similarity with the other known aminopyridine, namely tacrine, but is superior to the last in relation to the efficacy and safety (Kojima et al., 1998). Cholinesterases are enzymes that carry out the inactivation of acetylcholine. In turn, acetylcholine is the main neurotransmitter responsible for the conduction of excitement in the peripheral nervous system, maintenance of neuromuscular transmission, increased contractility and tone of smooth muscle organs; it also produces a stimulating effect on the central nervous system. Thus, the inhibition of cholinesterase activity leads to the maintenance of the acetylcholine level and corresponding activity of neuroregulatory functions.

It is known that the sexual function is controlled by neuronal, neuroendocrine, endocrine and neurotransmitter systems. The cholinergic system is involved in the sexual behavior via the M-cholinergic mechanism that transmits non-specific information to the neocortex from subcortical structures (reticular formation, hypothalamus) and in the functioning of the cortical "awakening" system. The cholinesterase inhibitors affect the neuroregulatory processes including those accompanying sexual behavior. For example, in vivo experiments demonstrated that the introduction of the cholinesterase inhibitor (eserine) into the lateral ventricle in the brain increases the lordosis in ovariectomized, hypoestrogenic rats (Clemens et al., 1989). The authors also demonstrate the influence of the other cholinesterase inhibitor, acetylcholine, to enhance the sexual receptivity of the animals. The performed experiments prove that sexual behavior is regulated, among others, via endogenous cholinergic activity.

Similar effects of enhancing sexual behavior in animals when administering cholinesterase inhibitors are demonstrated in a number of other experiments (Clemens et al., 1980; Dohanich et al., 1990; Menard & Dohanich, 1994; Dohanich & Clemens, 1981).

In vivo studies showed that against the background of different external stimuli, such as memorizing or learning, stress, any research, and other exposures that directly or indirectly affect the brain activity, the increase in the acetylcholine level is observed, and its level may vary depending on the type of external exposure.

(Mitsushima, 2010). The experiment demonstrated that against the background of sexual exposure, an increase in the level of acetylcholine occurs, therefore, this neurotransmitter performs one of the most important neuroregulatory functions influencing the sexual behavior and functions. It can be concluded that the inhibitors of cholinesterase (which negates the action of acetylcholine) are advisable to use for improvement of all components providing the neuroregulatory basis of sexual activity.

The indirect indication of the effect of cholinesterase inhibitors on the enhancement of sexual behavior is also side effects of the use of the drugs of this class in the treatment of patients with dementia. For example, when the drug donepezil (cholinesterase inhibitor) is given to patients in the treatment of Alzheimer's disease, an increase in the sexual behavior in these patients, even to the manifestation of sexual aggression is observed (Bianchetti et al., 2003; Bouman & Pinner, 1998; Lo Coco & Cannizzaro , 2010).

Thus, the regulation of the sexual behavior via cholinergic mechanisms is carried out in the central level. The clinical forms of sexual dysfunction are psychogenic and stressor disorders of the sexual function as well as disorders associated with the damage of the central and peripheral nervous system and associated with decreased levels of sex hormones. For this reason, the drugs from the group of cholinesterase inhibitors may be used in the treatment of the specified disorders.

### Brief description of drawings

Fig. 1 shows the effect of the drugs to be compared on the sexual activity (mean number of ejaculations) of hemigonadectomized rats when administering the drugs in mean therapeutic doses in the course of treatment.
Fig. 2 presents the effect of ipidacrine on the sexual activity of male rats using the model of chronic stress caused by electrical current exposure.
Fig. 3 shows the effect of ipidacrine on the sexual activity of rats using the model of spontaneous sexual dysfunction (by criterion of the number of ejaculations)

### Description of the preferred embodiments

### Definitions

The term "pharmaceutical formulation" means a formulation in tablet or capsule form (as an example but not limited to hard gelatin capsules), including that of prolonged action, containing ipidacrine as a main biologically active substance in an effective amount from 3 to 300 mg per dose and additionally containing the pharmaceutically acceptable excipients.

The term "pharmaceutically acceptable excipient" means a substance needed to improve tableting, such as, but not limited to, hydroxypropyl methylcellulose, microcrystalline cellulose, colloidal silicon dioxide, magnesium stearate and other conventional pharmaceutical excipients which do not limit the scope of the invention.

### Embodiments of invention

To illustrate the effects of pharmaceutical formulations based on ipidacrine, here in are the following examples which do not limit the scope of the invention. Example 1: Preparation of pharmaceutical formulations containing ipidacrine.

The pharmaceutical tableted formulations (including those of prolonged action) on the basis of ipidacrine are made in a standard way of direct compression. All the ingredients (except magnesium stearate) are stirred to obtain a homogeneous powder mixture using a Y-shaped mixer or similar equipment. Then magnesium stearate is added, and the resulting mixture is stirred for 2 minutes. The obtained tablet mass is subjected to a tableting process (tablet diameter of 10 mm or 11 mm in accordance with industry standard OST 64-072-89, with a scoreline and bevel edge) at a pressing force of 9 - 10 kN.

Pharmaceutical formulations in capsules are made using standard processing methods by mixing active ingredients and excipients in the correct proportion and further encapsulation.

### Pharmaceutical formulation 1 (content for 1 tablet of 100mg):

### Active substance:

Ipidacrine 3mg
*Pharmaceutically acceptable excipients:*
hydroxypropyl methylcellulose 35mg
microcrystalline cellulose 60mg
colloidal silicon dioxide 1mg
magnesium stearate 1 mg

### Pharmaceutical formulation 2 (content for 1 tablet of 250mg):

### Active substance:

Ipidacrine 40mg

### Pharmaceutically acceptable excipients:

hydroxypropyl methylcellulose 75mg
microcrystalline cellulose 128.74mg
colloidal silicon dioxide 3.13mg
magnesium stearate 3.13mg

### Pharmaceutical formulation 3 (content for 1 tablet of 600mg):

### Active substance:

Ipidacrine 150mg

### Pharmaceutically acceptable excipients:

hydroxypropyl methylcellulose 188mg
microcrystalline cellulose 250mg
colloidal silicon dioxide 6mg
magnesium stearate 6mg

### Pharmaceutical formulation 3 (content for 1 tablet of 1000mg):

### Active substance:

Ipidacrine 300mg

### Pharmaceutically acceptable excipients:

hydroxypropyl methylcellulose 280mg
microcrystalline cellulose 400mg
colloidal silicon dioxide 10mg
magnesium stearate 10mg

### Pharmaceutical formulation 4 (content for 1 tablet of 230mg):

### Active substance:

Ipidacrine 40mg

### Pharmaceutically acceptable excipients:

microcrystalline cellulose 140mg
colloidal silicon dioxide 2mg
magnesium stearate 2mg

### Pharmaceutical formulation 6 (content for 1 prolonged action tablet of 600mg)

### Active substance:

Ipidacrine 60mg

### Pharmaceutically acceptable excipients:

microcrystalline cellulose 216mg
colloidal silicon dioxide 2mg
hydroxypropyl methylcellulose 120mg
magnesium stearate 2mg

Example 2. Evaluation of the effects of ipidacrine pharmaceutical formulation in rats with the reduced sexual activity caused by hemigonadectomy. The hemigonadectomy is similar to the pathological processes observed in the clinical picture and associated with the decrease in hormone production by the sex glands. The model of hypogonadal state in rats induced by the hemigonadectomy corresponds to the pathological processes observed in the clinical picture and associated with the decreased hormone production of the sex glands.

The study of the ipidacrine pharmacological activity was conducted in albino male rats. The following six groups of animals were formed: 1 - intact, 2 - treated with sildenafil, 3 - galantamine, 4 - ipidacrine, 5 - gonadectomized, 6 - control. 30 days before the start of the main study, the experiment was performed (3 times a week for 2 weeks), namely introduction of a receptive female to the male rat. The number of ejaculations was recorded. Those animals whose level of sexual activity was characterized by stable performance of 1-2 ejaculations during the period of the test were selected.

The selected male rats underwent the hemigonadectomy (at the right) performed under ether anesthesia. Then, during 7 and 14 days prior to the course administration of the drugs, daily testing of sexual activity of the hemigonadectomized animals was carried out. The latency period and the number of mounts, intromissions and ejaculations in different groups of the animals were considered.

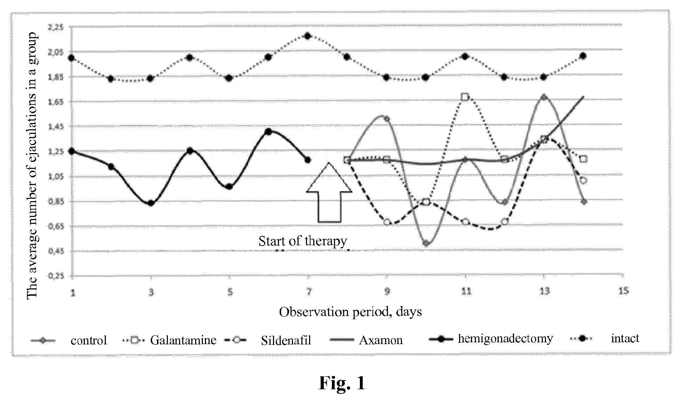

Administration of ipidacrine at a dose of 1.7 mg/kg once daily for 7 days to hemigonadectomized rats eliminates fluctuations in the level of the sexual activity due to external stressor effects throughout the whole course of the therapy and contributes to the increase of central motivation and ejaculatory components. Ipidacrine in the course therapy of 2 times a day for 14 days in hemigonadectomized rats at a dose range of 0.85 to 5.1 mg/kg produces a dose-dependent increase in the sexual activity of the animals, with a maximum manifestation in 3 to 5 days at a dose of 5.1 mg/kg, and in 11 to 14 days at a dose of 0.85 to 1.7 mg/kg (Fig. 1).

Example 3. Evaluation of the effects of an ipidacrine pharmaceutical formulation at reduced sexual activity in rats caused by physical stress.

The most appropriate model (in terms of the ease of implementation) is the model of psychogenic sexual dysfunction caused by exposure to physical factors (electrical current) in rats.

The pre-experiment with the animals was performed similar to that described in Example 2.

The two groups of the animals similar in the sexual activity level were formed: control (administration of distilled water), pharmaceutical formulation (on ipidacrine basis at a dose of 1.7 mg/kg). The animals of the control and experimental groups were daily exposed to current with a voltage of 30V for 30 min once a minute with an impulse with duration of 1 sec. The drug was administered 30 minutes before the stress, the testing was performed 60 min after the stress.

The administration of ipidacrine at a dose of 1.7 mg/kg once a day prior the stress contributed to a slower formation of sexual dysfunction in the rats. In an initial period (1 to 3 days), the parameters of activity were comparable to the parameters in the group nonsusceptible to the stress. Starting from the 5^{th} day, a significant increase in the latency period of mounts and intromissions compared to the animals nonsusceptible to stress (in the group without treatment, such changes were observed from the 1^{st} day of the experiment) was registered. Significant differences from the group without treatment on a number of indicators were observed on the 7^{th} day (latency period of intromissions) and on the 10^{th} day (latency period of intromissions and mounts).

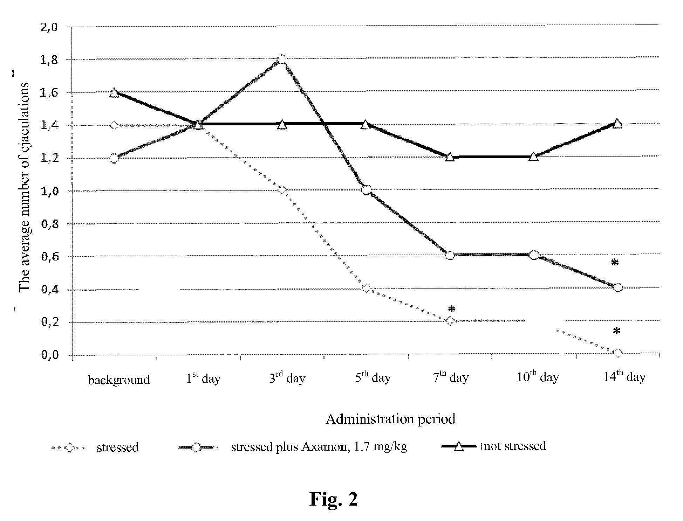

When comparing the sexual activity of the stressed animals without treatment and against the background of the ipidacrine therapy at a dose of 1.7 mg/kg, the period from the 3^{rd} to 10^{th} day of the experiment was the most significant. The administration of ipidacrine provided the maintenance of the frequency of ejaculations at a level of 60-100%, while in the comparison group, this figure reduced to 20%. In the same period, a higher rate of renewal of activity after the first ejaculation was recorded in the animals of the ipidacrine group. By the end of the 14 day exposure to stress, the number of ejaculations remained at a level of 0.4-0.6 (on the 10^{th} and 14^{th} day) in the animals of the treatment group, while in the group without treatment, this parameter was not greater than 0.2 (7^{th} and 14^{th} day) (Fig. 2).

Example 4. The evaluation of effects of ipidacrine pharmaceutical formulation on a model of initially hypoactive male rats with spontaneously reduced sexual function. This model is associated with the clinical conditions of anorgasmia or delayed ejaculation in humans. According to the results of the five-fold testing of the intact animals, the groups of hypoactive male rats were formed. The criterion for selection was average number of ejaculations of less than 0.5 according to the results of the five-fold testing.

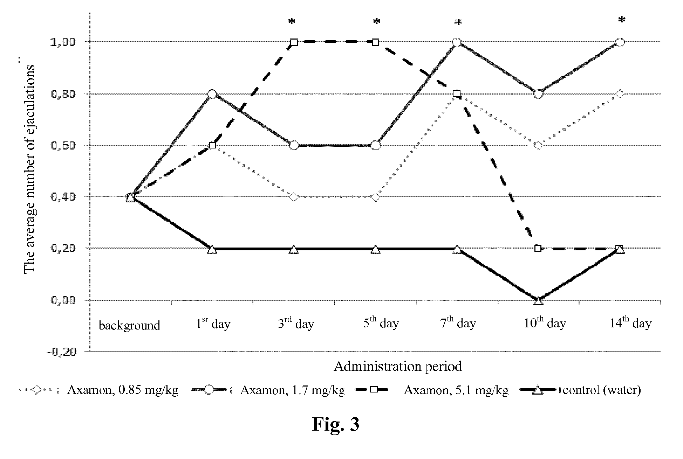

To assess the effect of ipidacrine on sexual function of rats with the reduced sexual activity, the groups of the animals with initially low manifestations of copulatory and ejaculatory components of behavior were formed. This model reflects the clinical conditions of anorgasmia and delayed ejaculation. The effect of the drug on the integral indicator of the sexual function (number of ejaculations) at a dose of 0.85 to 5.1 mg/kg when administered daily is shown in Fig. 3

In hypoactive animals undergoing daily course therapy with the pharmaceutical composition (ipidacrine content of 0.85 mg/kg), an increase in the sexual activity (starting with the 2^{nd} week) was registered. It was manifested in the reduction of the latency period of mounts and intromissions (significant differences from the background were registered on the 7^{th} to 14^{th} day of observation). These data allow us to characterize the effect of the drug (0.85 mg/kg) on the trend level as similar changes of indicators were observed in the control group. An increase in the average number of ejaculations in the group up to 0.6-0.8 and increase of the rate of occurrence of ejaculations to 80% were noted. In some animals, the renewal of sexual activity was observed after the first ejaculation (on the average in 4-6 minutes) in contrast to the control group. While administering the pharmaceutical formulation with ipidacrine at a dose of 1.7 mg/kg (considering interspecies dose conversion), an increased central motivational component (2 to 4 -fold reduction in the latency time of mounts and intromissions) after the first administration of the drug was revealed. In the subsequent periods, a slight decrease in the sexual activity (on the 3^{rd} or 5^{th} day) with an increase of indicators of sexual behavior on the 7^{th}, 10^{th} and 14^{th} day of the administration of the drug was observed. This was accompanied by an increase of the ejaculatory component in comparison with the control throughout the course of administration of the drug in the test dose. The number of animals able to ejaculate also increased (up to 80%). The administration of ipidacrine at a higher dose (5.1 mg/kg) increased the sexual activity in the animals during the first 5 days of observation, which was manifested by an increase in central motivational and ejaculatory components. The latency period of mounts reduced from 33.0±5.35 to 9.3±1.93 s (p <0.05), and the latency period of intromissions from 59.8 ±11.34 to 10.5 ± 1.76 (p <0.05) in 3 days of observation. Subsequent administration of the drug at this dose produced a negative effect on the manifestation of the sexual activity while in 10, 14 days of the experiment the values of indicators were comparable to the control.

Therefore, the administration of ipidacrine in the animals with the spontaneous sexual hypoactivity leads to an increase of copulatory and ejaculatory components when administered at doses of 1.7 and 5.1 mg/kg. The drug at a dose of 5.1 mg/kg is effective only when administered for 5 days. Ipidacrine at a dose of 1.7 mg/kg showed an activating effect on the sexual function throughout the whole observation period (14 days).

### References:

- Clemens LG, Barr P, Dohanich GP. Cholinergic regulation of female sexual behavior in rats demonstrated by manipulation of endogenous acetylcholine. Physiol Behav. 1989 Feb;45(2):437-42
- Dohanich GP, McMullan DM, Brazier MM. Cholinergic regulation of sexual behavior in female hamsters. Physiol Behav. 1990 Jan;47(1):127-31.
- Menard CS, Dohanich GP. Estrogen dependence of cholinergic systems that regulate lordosis in cycling female rats. Pharmacol Biochem Behav. 1994 Jun;48(2):417-21.
- Clemens LG, Humphrys RR, Dohanich GP. Cholinergic brain mechanisms and the hormonal regulation of female sexual behavior in the rat. Pharmacol Biochem Behav. 1980 Jul;13(1):81-8
- Dohanich GP, Clemens LG. Brain areas implicated in cholinergic regulation of sexual behavior. Horm Behav. 1981 Jun;15(2):157-67.
- Mitsushima D. Sex steroids and acetylcholine release in the hippocampus. Vitam Horm. 2010;82:263-77
- OCT64-072-89.
- Bianchetti A, Trabucchi M, Cipriani G. Aggressive behaviour associated with donepezil treatment: a case report. Int J Geriatr Psychiatry. 2003 Jul;18(7):657-8.
- Bouman WP, Pinner G. Violent behavior-associated with donepezil. Am J Psychiatry. 1998 Nov;155(11):1626-7.
- Lo Coco D, Cannizzaro E. Inappropriate sexual behaviors associated with donepezil treatment: a case report. J Clin Psychopharmacol. 2010 Apr;30(2):221-2.
- Kojima J, Onodera K, Ozeki M, Nakayama M. Ipidacrine (NIK-247): A Review of Multiple Mechanisms as an Antidementia Agent CNS Drug Reviews 1998, Vol. 4, No. 3, pp. 247.259

## Claims

1. Ipidacrine for use in the treatment of potency disorders.

2. Ipidacrine for use in the treatment of potency disorders according to claim 1, **characterized in that** said potency disorders are selected from the group of disorders of sexual activity: disorders associated with the decreased production of hormones by the sex glands, disorders caused by chronic stress (including physical one), disorders that are manifested against the background of spontaneously reduced sexual function, including manifested anorgasmia or delayed ejaculation.

3. Ipidacrine for use in the treatment of potency disorders according to claim 1 or 2, **characterized in that** ipidacrine is formulated in a pharmaceutical formulation in the amount of 3 to 300 mg per dose.

4. Ipidacrine for use in the treatment of potency disorders according to claim 3, **characterized in that** said pharmaceutical formulation additionally contains pharmaceutically acceptable excipients selected from the following group: hydroxypropyl methylcellulose, microcrystalline cellulose, colloidal silicon dioxide, magnesium stearate or their combinations.

5. Ipidacrine for use in the treatment of potency disorders according to claim 3 or 4, **characterized in that** said pharmaceutical formulation is a tablet or prolonged action tablet or a hard gelatin capsule.

## Patentansprüche

1. Ipidacrin für dessen Verwendung bei der Behandlung von Potenzstörungen.

2. Ipidacrin für dessen Verwendung bei der Behandlung von Potenzstörungen nach Anspruch 1, **dadurch gekennzeichnet, dass** die genannten Potenzstörungen aus der Gruppe von Störungen der sexuellen Aktivität ausgewählt werden: Störungen, welche mit der verringerten Hormonproduktion durch die Keimdrüsen assoziiert sind, Störungen, welche durch chronischen Stress (einschließlich des körperlichen Stresses) hervorgerufen werden, Störungen, welche gegenüber dem Hintergrund von einer spontan verringerten Sexualfunktion manifest werden, einschließlich der manifesten Anorgasmie oder der verzögerten Ejakulation.

3. Ipidacrin für dessen Verwendung bei der Behandlung von Potenzstörungen nach Anspruch 1 oder 2, **dadurch gekennzeichnet, dass** das Ipidacrin in einer pharmazeutischen Formulierung in einer Menge von 3 bis 300 mg per Dosis formuliert ist.

4. Ipidacrin für dessen Verwendung bei der Behandlung von Potenzstörungen nach Anspruch 3, **dadurch gekennzeichnet, dass** die genannte pharmazeutische Formulierung zusätzlich pharmazeutisch akzeptable Hilfsstoffe, ausgewählt aus der folgenden Gruppe, enthält: Hydroxypropylmethylcellulose, mikrokristalline Cellulose, kolloidales Siliciumdioxid, Magnesiumstearat oder Kombinationen derselben.

5. Ipidacrin für dessen Verwendung bei der Behandlung von Potenzstörungen nach Anspruch 3 oder 4, **dadurch gekennzeichnet, dass** die genannte pharmazeutische Formulierung eine Tablette oder Tablette mit verlängerter Wirkung oder eine harte Gelatinekapsel ist.

## Revendications

1. Ipidacrine pour son utilisation dans le traitement des troubles de puissance sexuelle.

2. Ipidacrine pour son utilisation dans le traitement des troubles de puissance sexuelle selon la revendication 1, **caractérisée en ce que** lesdits troubles de puissance sexuelle sont choisis parmi le groupe des troubles d'activité sexuelle ; des troubles associés à la production réduite d'hormones par les glandes sexuelles, des troubles produits par le stress chronique (y compris le stress physique), des troubles qui se manifestent à l'encontre de la fonction sexuelle réduite spontanément, y compris l'anorgasmie manifestée ou l'éjaculation retardée.

3. Ipidacrine pour son utilisation dans le traitement des troubles de puissance sexuelle selon la revendication 1 ou 2, **caractérisée en ce que** l'ipidacrine est formulée sous forme de formulation pharmaceutique en une quantité allant de 3 à 300 mg par dose.

4. Ipidacrine pour son utilisation dans le traitement des troubles de puissance sexuelle selon la revendication 3, **caractérisée en ce que** ladite formulation pharmaceutique contient en outre des excipients pharmaceutiquement acceptables choisis parmi le groupe suivant : hydroxypropyle méthycellulose, cellulose microcristalline, dioxyde de silicium colloïdal, stéarate de magnésium ou leurs combinaisons.

5. Ipidacrine pour son utilisation dans le traitement des troubles de puissance sexuelle selon la revendication 3 ou 4, caractérisée en ce ladite formulation pharmaceutique est un comprimé ou un comprimé à action prolongée ou une gélule de gélatine dure.
